# EUROPÄISCHE PATENTSCHRIFT

(11) **EP 1 382 465 B1**
(45) Veröffentlichungstag und Bekanntmachung des Hinweises auf die Patenterteilung: **02.02.2005**
(21) Anmeldenummer: 02016094.1
(22) Anmeldetag: 19.07.2002
(51) Int. Cl.: B43L 23/08

(54) **Spitzer für Weichminenstifte**
Sharpener for soft-core pencils
Taille-crayon pour crayons à mine tendre

(43) Veröffentlichungstag der Anmeldung: 21.01.2004
(73) Patentinhaber: KUM Limited, Trim, Co. Meath (IE)
(72) Erfinder: Lüttgens, Fritz Dr., 91054 Erlangen (DE)
(74) Vertreter: Tergau & Pohl Patentanwälte

(56) Entgegenhaltungen:
- DE-U- 29 723 492
- US-A- 4 996 800

## Beschreibung

Die Erfindung bezieht sich auf einen Spitzer für Weichminenstifte mit einem Spitzergehäuse und mit Formgebungsmitteln, insbesondere zur Verwendung für medizinische oder pharmazeutische Stifte.

Bei einem Spitzer für Weichminenstifte sind üblicherweise als Formgebungsmittel ein Spitzmesser und ein Minenformer vorgesehen. Ein derartiger Spitzer wird insbesondere zur Formgebung von Weichminenstiften, wie etwa von Schmink- oder Kosmetikstiften, eingesetzt. Beim Spitzen wird der Stift innerhalb eines Führungskanals gegen die Schneide des Spitzmessers gedreht. Dadurch hebt die Schneide des Spitzmessers einen Schälspan von der Minenspitze des Stiftes und gegebenenfalls gleichzeitig vom Minenmantel ab. Um die Minenspitze des Stiftes wahlweise zur Konturenschärfe relativ spitz oder abgerundet zu formen, ist der Spitzer darüber hinaus häufig mit einem Minenformer als weiteres Formgebungsmittel versehen.

Im Bereich der Anwendung von Spitzern für Stifte, deren Minen für den direkten Kontakt mit menschlicher Haut gedacht sind, sind beispielsweise Aspekte der Hautverträglichkeit oder sogar der Hautpflege besonders zu beachten und zu berücksichtigen. Um unerwünschte Hautirritationen oder ―schäden zu vermeiden, sind die Stifte, insbesondere hinsichtlich der chemischen Zusammensetzung ihrer Minen, geeignet gewählt. Dabei sind neben der eigentlichen ursprünglichen Zusammensetzung der Minen aber auch nachträgliche Reaktionen zu berücksichtigen. Diese gehen beispielsweise mit der chemischen Zersetzung der Minenmaterialien aus Altersgründen einher oder resultieren aus späteren Verunreinigungen. Daher sind an ihre Spitzer im Gegensatz zu Spitzern beispielsweise für Bleistifte vergleichsweise hohe Anforderungen an die Hygiene zu stellen.

Aus diesen Gründen können derartige Spitzer für eine bedarfsweise mögliche Reinigung ihrer wesentlichen Funktionskomponenten besonders ausgelegt sein. Um eine Entfernung der abgehobenen Weichminenmasse ohne Beschädigung der Spitzmesserschneide oder des Minenformers zu gewährleisten, ist aus den Druckschriften DE 297 23 492 U1 sowie EP 1 043 173 A1 eine Ausstattung des Spitzers für Kosmetikstifte mit einem zusätzlichen lösbarem Reinigungsstäbchen und eine Kombination des Minenformers für Kosmetikstifte mit einer Reinigungsvorrichtung bekannt.

Allerdings sind im medizinischen oder pharmazeutischen Bereich generell besonders hohe Maßstäbe bei den Hygieneanforderungen anzulegen. So werden üblicherweise bei der Verabreichung von Medikamenten oder im Zusammenhang mit therapeutischen Maßnahmen "Einmal-Materialien", wie beispielsweise bei Handschuhen, Spritzen, Auftragstäbchen etc., verwendet, oder es werden schnell und gründlich zu reinigende Materialien, wie beispielsweise geeignete Metalle oder Kunststoffe, gebraucht. Im Gegensatz dazu ist in diesen Bereichen der Einsatz von Weichminenstiften nicht ohne weiteres möglich, obwohl deren Verwendung aus Gründen der einfachen Handhabbarkeit wünschenswert sein kann.

Der Erfindung liegt daher die Aufgabe zugrunde, einen Spitzer der oben genannten Art anzugeben, der die Verwendung von Weichminenstiften auch im medizinischen oder pharmazeutischen Bereich in besonderem Maße begünstigt.

Diese Aufgabe wird erfindungsgemäß gelöst, indem der Spitzer mit einem Spitzergehäuse und mit Formgebungsmitteln ausgestattet ist, welche mit einem Reinigungsmittel zur Desinfektion eines zu spitzenden medizinischen oder pharmazeutischen Stiftes versetzt sind.

Die Erfindung geht dabei von der Überlegung aus, dass medizinische oder pharmazeutische Stifte mit Rezepturen oder Ingredienzien, die beispielsweise eine antibakterielle, antiviröse oder fungizide Wirkung entfalten, einer speziellen Sorgfalt und Handhabung hinsichtlich der Hygiene bedürfen. Die Behandlung insbesondere verletzter oder erkrankter Hautpartien mit einem medizinischen oder pharmazeutischen Stift kann nämlich bei direktem Kontakt zu Kontaminationen des Stiftes oder seines Minenmaterials führen. Um einen erneuten Gebrauch des Stiftes ohne Gefährdung für den Verwender zu ermöglichen, sollte demzufolge eine zuverlässige Reinigung in regelmäßigen Intervallen vorgenommen werden. Dies ist auf besonders einfache Weise möglich, indem der Reinigungsvorgang mit anderen ohnehin erforderlichen Bearbeitungstechniken für den Stift zusammengefasst wird. Dazu ist vorgesehen, den Stift gleichzeitig mit einer eventuell erforderlich werdenden Konturierung seiner Mine einer solchen Reinigung zu unterziehen. Um dies zu gewährleisten, sind diejenigen Komponenten des Spitzers, die beim Spitzen in unmittelbarem Kontakt mit der je nach Anwendungsgebiet mehr oder weniger stark bakteriell-, virös- oder fungös-beladenen Oberfläche des medizinischen oder pharmazeutischen Stiftes treten, entsprechend ausgebildet. Daher sind die Formgebungsmittel des Spitzers mit einem Reinigungsmittel zur Desinfektion eines zu spitzenden medizinischen oder pharmazeutischen Stiftes versetzt.

Bevorzugte Ausgestaltungen des erfindungsgemäßen Spitzers und insbesondere seiner Formgebungsmittel sind in den Unteransprüchen beschrieben.

Um die Oberfläche des medizinischen oder pharmazeutischen Stiftes chemisch zu desinfizieren, ist das Reinigungsmittel vorzugsweise als Zusatzstoff dem Basisstoff einer Anzahl von Komponenten der Formgebungsmittel beigemischt. Damit wird aktiv das Wachstum von Krankheitserregern auf der Mine des Stiftes behindert und eine Infizierung der zu behandelnden Partien vermieden.

Abhängig von der gewünschten Art und Dauer des einzusetzenden Reinigungsmittels wird der Basisstoff einer Anzahl von Komponenten der Formgebungsmittel, vorzugsweise ein Kunststoff, schon bei seiner Herstellung mit entsprechenden chemischen Zusätzen versetzt, oder das Reinigungsmittel wird erst später als ein- oder mehrlagige Beschichtung auf einer Anzahl von Komponenten der Formgebungsmittel aufgebracht.

Durch das mechanische Spitzen eines medizinischen oder pharmazeutischen Stiftes wird die je nach Anwendungsgebiet mehr oder weniger stark mit Krankheitserregern beladene Oberfläche entfernt und somit die medizinisch- oder pharmazeutisch-aktive Minenmasse regeneriert. Beim Spitzer hingegen bleibt das beim Abtragen belastete und zur Verklebung neigende Weichminenmaterial haften. Folglich sollte eine Reinigung des Spitzers vorgenommen werden, um eine Infizierung anderer Stifte oder eine sich ständig abwechselnde Ansteckung zwischen ein- und demselben Stift und Spitzer oder sogar eine akkumulierende Verschmutzung zu vermeiden. Damit das belastete und klebende Weichminenmaterial beim Abtragen durch den Spitzer nicht an der Oberfläche des Spitzers haften bleibt und ihn somit verunreinigt, sollten die Formgebungsmittel - und zweckmäßigerweise auch zumindest die mit dem Weichminenmaterial in Kontakt tretenden Teile oder Komponenten des Spitzers, insbesondere die entsprechenden Gehäuseteile - mit einer spezifische Verunreinigungen abweisenden Oberfläche versehen und somit passiv reinigend wirksam sein.

Für eine solche zusätzlich oder alternativ zur aktiven Reinigung vorgesehenen passiven Reinigungsmaßnahme ist auf einer Anzahl von Komponenten der Formgebungsmittel vorzugsweise eine Beschichtung, die Polytetrafluorethylen aufweist, aufgebracht. Polytetrafluorethylen (PTFE) ist ein chemisch und biologisch inerter sowie hydrophober Kunststoff, der unter dem Namen Teflon im Handel ist. Bedingt durch den zudem extrem niedrigen Reibungskoeffizienten von Polytetrafluorethylen findet dieser Kunststoff als Anti-Haft-Beschichtung vielfältige Einsatzmöglichkeiten. Somit wird durch eine entsprechende Beschichtung eines Formgebungsmittels die schmutzabweisende Wirkung dieses Systems ausgenutzt. Diese Beschichtung führt durch die quasi selbstreinigende Wirkung zur Minimierung der Verunreinigung des Spitzers und somit gleichzeitig zur Verminderung einer erneuten Belastung der durch das Spitzen frisch hervorgebrachten medizinisch- oder pharmazeutisch-aktiven Minenmasse mit Bakterien, Viren, Fungi oder dergleichen.

Zum selben Zweck ist alternativ oder kombiniert zwechmäßigerweise eine Beschichtung auf zumindest einem Formgebungsmittel vorgesehen, die im Bereich ihrer Oberfläche eine Lotusstruktur aufweist. Eine Lotusstruktur bewirkt einen sog. Lotuseffekt. Die Lotusstruktur ist eine mikroskopische Noppenstruktur, die nach dem Lotusblumenblatt benannt ist. Diese Struktur sorgt dafür, dass Wasser und Schmutz "abperlen", was als Lotuseffekt bezeichnet wird. Dadurch entfällt ein aufwendiges Reinigen.

Zur chemisch und damit aktiv reinigenden Bearbeitung und Desinfektion der Oberfläche des medizinischen oder pharmazeutischen Stiftes umfasst der Zusatzstoff bzw. die Beschichtung auf einer Anzahl von Komponenten der Formgebungsmittel vorzugsweise einen migrierenden Stoff. Das bedeutet, dass insbesondere antibakteriell wirkende Stoffe, wie beispielsweise Silberionen, aus den Formgebungsmitteln dauerhaft und gleichmäßig heraustreten können. Somit wird das Minenmaterial des medizinischen oder pharmazeutischen Stiftes beim Spitzvorgang ständig mit einem Reinigungsmittel mit antimikrobieller Wirkung beaufschlagt.

Damit der medizinische oder pharmazeutische Stift beispielsweise seine antibakterielle, antiviröse oder fungizide Wirkung optimal entfalten kann, werden zur Desinfektion der Minenmasse des Stiftes und zur Reinhaltung der Komponenten der Formgebungsmittel des Spitzers die Reinigungsmittel nach Möglichkeit untereinander kombiniert und bedarfsweise auf einzelne oder alle Komponenten verteilt.

Als Komponenten weisen die Formgebungsmittel vorteilhafterweise ein Spitzmesser und einen Minenformer auf. Diese können also beide oder jeder für sich mit einem Reinigungsmittel versetzt oder beschichtet werden.

Um die Reinigungsmöglichkeiten zu erweitern, ist eine Ausweitung der Einsatzbereiche durch Übertragung des obigen Konzepts auf andere Teile des Spitzers, beispielsweise das Spitzergehäuse vorteilhaft. Das Spitzergehäuse ist dabei mit dem Reinigungsmittel versetzt, das beispielsweise als Anti-Haft-Beschichtung fungiert oder desinfizierend wirkende Chemikalien abgibt.

Die mit der Erfindung erzielten Vorteile bestehen insbesondere darin, dass durch die Ausstattung der Formgebungsmittel mit einem Reinigungsmittel eine in besonderem Maße für medizinische oder pharmazeutische Stifte und deren Spitzer adäquate Reinigung ermöglicht ist. Dies begünstigt besonders die Verwendung eines derartig ausgebildeten Spitzers für Weichminenstifte selbst in Bereichen mit hohen Hygieneanforderungen. Gerade durch den Einsatz des Reinigungsmittels als Zusatzstoff für den Basisstoff einer Anzahl von Komponenten der Formgebungsmittel oder als ein- oder mehrlagige Beschichtung auf einer Anzahl von Komponenten der Formgebungsmittel ist eine bedarfsweise Desinfektion der Oberfläche der Stifte bzw. eine Reinhaltung der Formgebungsmittel als solche möglich. Durch die bedarfsweise Verwendung eines migrierenden Stoffes, von Polytetrafluorethylen oder einer Beschichtung mit Lotusstruktur ist ein besonders effektiver Einsatz der physikochemischen Eigenschaften für die Desinfektion bzw. Reinigung möglich. Ferner können außer den Formgebungsmitteln auch andere Teile des Spitzers, vorzugsweise das Spitzergehäuse, mit einem solchen Reinigungsmittel versetzt werden, um die Einsatzmöglichkeiten zu erweitern.

Ein Ausführungsbeispiel der Erfindung wird im Folgenden anhand einer Zeichnung näher erläutert. Darin zeigen:
- Fig. 1: eine perspektivische Draufsicht auf einen Spitzer mit Sicht auf die Stift-Einführungsseite,
- Fig. 2: eine perspektivische Draufsicht auf einen Spitzer mit Sicht auf die Spitzergehäuse-Rückwand,
- Fig. 3: eine Draufsicht auf die Spitzergehäuse-Oberseite, und
- Fig. 4a-c: je eine schematische Darstellung eines Minenformers mit verschiedenen Reinigungsmitteln.

Gleiche Teile sind in allen Figuren mit denselben Bezugszeichen versehen.

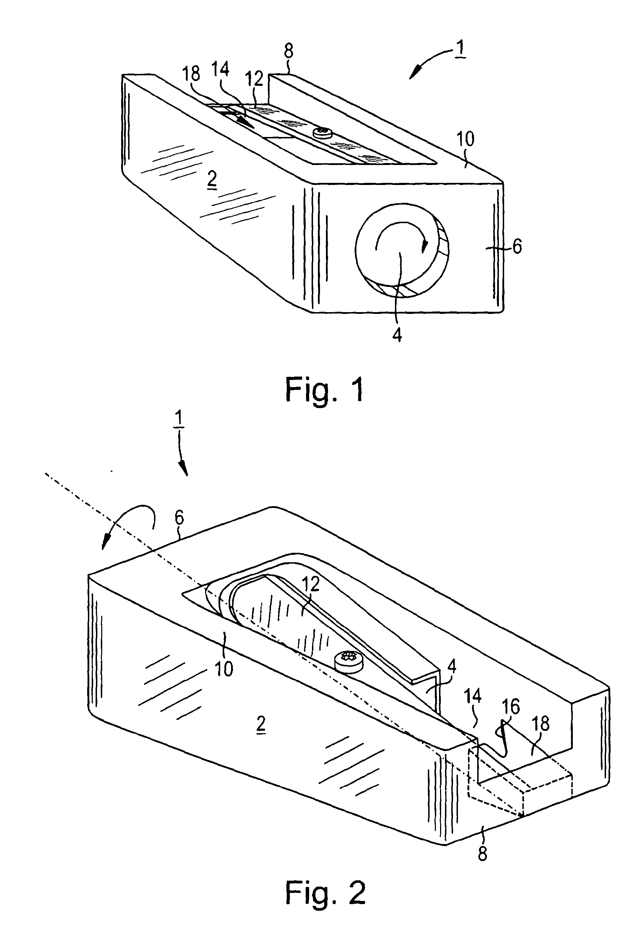

Der Spitzer 1 gemäß den Figuren 1, 2 und 3 weist ein Spitzergehäuse 2 mit einem Führungskanal 4 für einen zu spitzenden, hier nicht dargestellten Stiftkonus auf und umfasst eine Stift-Einführungsseite 6, eine Spitzergehäuse-Rückwand 8 und eine Spitzergehäuse-Oberseite 10. Tangential zum Führungskanal 4 trägt das Spitzergehäuse 2 ein schraubfixiertes Spitzmesser 12. Der Führungskanal 4 läuft von der Stift-Einführungsseite 6 des Spitzergehäuses 2 sich konisch verjüngend in Richtung auf einen den Stiftkonus aufnehmenden und unterhalb des vorderen Endes des Spitzmessers 12 positionierten Freiraum 14 des Spitzergehäuses 2 aus. Beim Spitzen wird der Stift innerhalb des Führungskanals 4 in Spitzrichtung, die in den Figuren 1 und 2 durch Pfeile angedeutet ist, gegen die Schneide des Spitzmessers 12 gedreht. Dadurch hebt die Schneide des Spitzmessers 12 einen Schälspan von der Minenspitze des Stiftes und gegebenenfalls gleichzeitig vom Minenmantel ab. Um die Minenspitze des Stiftes wahlweise zur Konturenschärfe relativ spitz oder zur Vermeidung von Verletzungen abgerundet zu formen, steht in den Freiraum 14 ein mit einer Formkante 16 versehener Minenformer 18 hinein. Dieser Minenformer 18 stellt beispielsweise ein als Fassonmesser oder Schaberippe ausgestaltetes, vorzugsweise aus dem Spitzergehäuse 2 entnehmbares Einsatzteil dar. Dieses ist in Achsrichtung des Führungskanals 4 innerhalb des Freiraums 14 des Spitzergehäuses 2 verstellbar, insbesondere verschiebbar oder in unterschiedlichen Axialabständen zum Führungskanal 4 verschnappbar oder verrastbar.

Der Spitzer 1 ist in besonderem Maße für eine Verwendbarkeit auch für medizinische oder pharmazeutische Weichminenstifte ausgestaltet. Dafür ist der Spitzer 1 derart ausgebildet, dass er den im medizinischen oder pharmazeutischen Bereich bestehenden hohen Hygieneanforderungen Rechnung trägt. Diese implizieren nämlich eine außerordentlich sorgfältige Reinhaltung von in diesem Bereich einzusetzenden medizinischen oder pharmazeutischen Weichminenstiften, zu der der Spitzer 1 in besonderem Maße beiträgt. Zu diesem Zweck ist der Spitzer 1 für eine regelmäßige und zuverlässige Reinigung der einzusetzenden Stifte ausgelegt. Dazu sind die Formgebungsmittel des Spitzers 1 mit einem Reinigungsmittel zur Desinfektion des zu spitzenden medizinischen oder pharmazeutischen Stiftes versetzt. Dabei umfassen die Formgebungsmittel im Ausführungsbeispiel als Komponenten das Spitzmesser 12 und den Minenformer 18. Andere Teile des Spitzers 1, wie beispielsweise das Spitzergehäuse 2, können ebenfalls mit einem geeigneten Reinigungsmittel versetzt sein.

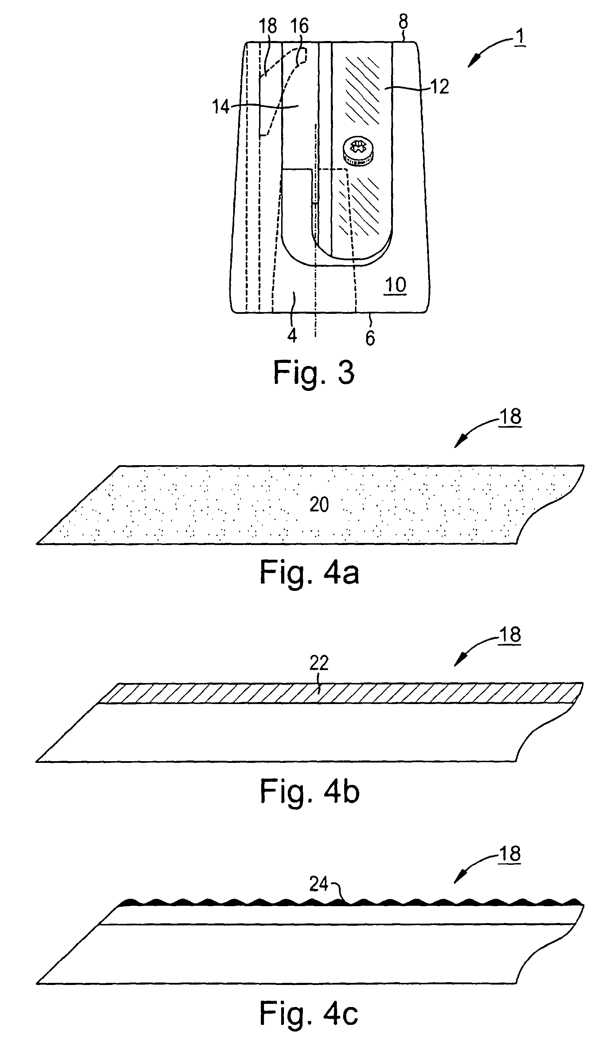

Unterschiedliche Ausgestaltungsmöglichkeiten hinsichtlich des Einsatzes verschiedener Reinigungsmittel sind beispielhaft in den Figuren 4a, b und c dargestellt. Darin ist jeweils nur eine Komponente der Formgebungsmittel, nämlich der Minenformer 18, gezeigt. Der Minenformer 18 ist aus hygienischen Gründen auf Polymerkunststoffbasis hergestellt. Der Minenformer kann auch eine metallische Komponente sein oder eine solche enthalten. Ebenso können die weiteren Komponenten des Spitzers 1, insbesondere das Spitzergehäuse 2, metallisch oder als spritzfähiges Holzderivat ausgeführt sein. Die dargestellten Ausführungsmöglichkeiten können aber selbstverständlich auch für andere Teile des Spitzers 1 zum Einsatz kommen.

Beim Minenformer 18 gemäß Figur 4a ist - wie durch die gepunktete Darstellung angedeutet - dem Basisstoff des Minenformers 18 das Reinigungsmittel als Zusatzstoff beigemischt. Dabei umfasst der Zusatzstoff einen migrierenden Stoff 20. Unter der Migration eines Stoffes ist insbesondere zu verstehen, dass er dauerhaft und gleichmäßig aus einem Basisstoff oder Trägermaterial herauswandert. Durch die Ausstattung des Zusatzstoffes mit einem migrierenden Stoff 20 ist gewährleistet, dass die Oberfläche des Minenformers 18 nahezu permanent mit den Anteilen oder Partikeln des Stoffes 20 benetzt ist. Der migrierende Stoff 20 kann folglich zu Desinfektionszwecken für den mit ihm beim Spitzen regelmäßig in Kontakt tretenden Stift oder dessen Mine herangezogen werden. Demnach ist der migrierende Stoff 20 im Ausführungsbeispiel gleichzeitig als desinfizierend oder antimikrobiell wirkender Stoff ausgestaltet. Dazu sind beispielsweise Silberionen eingesetzt. Ein solcher Stoff wandert aus dem Basisstoff heraus und tritt beim Spitzen in Kontakt mit der Oberfläche des zu spitzenden medizinischen oder pharmazeutischen Stiftes oder dessen Mine und entfaltet dort seine antibakterielle Wirkung.

Durch das mechanische Spitzen eines medizinischen oder pharmazeutischen Stiftes wird die bakteriell, virös, fungös oder mit anderen Krankheitserregern beladene Oberfläche entfernt und somit die medizinisch- oder pharmazeutisch-aktive Minenmasse regeneriert. Um dieses frische Minenmaterial dabei nicht wieder mit dem abgetragenen belasteten Weichminenmaterial über den Minenformer 18 in Kontakt zu bringen, ist eine spezifische Verunreinigungen abweisende Oberfläche des Minenformers 18 zweckmäßig. Dazu weist das Spitzmesser 12 quasi zur Selbstreinigung eine entsprechende Beschichtung 22, 24 auf.

Unter gezieltem Rückgriff auf die Materialeigenschaften von Polytetrafluorethylen ist daher in einer weiteren Ausführungsform auf dem Minenformer 18 eine auf diese Anforderungen zugeschnittene Beschichtung 22 aufgebracht. In diese in Figur 4b schraffiert dargestellte Anti-Haft-Beschichtung mit Polytetrafluorethylen können weder Wasser noch Schmutz auch nur teilweise eindringen. Damit ist physikochemisch ein Haftenbleiben selbst von zur Verklebung neigendem Weichminenmaterial am Minenformer 18 unterbunden.

Zum selben Zweck ist in einer alternativen Ausführungsform eine Beschichtung 24 auf dem Minenformer 18 vorgesehen, die im Bereich der Oberfläche eine in Figur 4c durch Wellen angedeutete Lotusstruktur aufweist. Diese mikroskopische Noppenstruktur sorgt physikalisch für ein "Abperlen" von Schmutz und Wasser, den sog. Lotuseffekt. Dadurch wird eine Verunreinigung des Minenformers 18 vermieden und somit gleichzeitig eine erneute Belastung der durch das Spitzen frisch hervorgebrachten medizinisch- oder pharmazeutisch-aktiven Masse mit Bakterien oder dergleichen gemindert.

Die Art der Reinigungsmittel sowie die Auswahl der Teile des Spitzers 1, die mit ihnen versetzt sind, können selbstverständlich in allen denkbaren Möglichkeiten gewählt und miteinander kombiniert werden. Zur Desinfektion der Minenmasse des Stiftes und zur Reinhaltung der Teile des Spitzers 1 sind die Reinigungsmittel folglich bedarfsweise auf einzelne oder alle Teile des Spitzers 1, wie Formgebungsmittel und Spitzergehäuse 2, verteilt, damit der medizinische oder pharmazeutische Stift beispielsweise seine antibakterielle, antiviröse oder fungizide Wirkung weitgehend unbeeinträchtigt entfalten kann.

### Bezugszeichenliste

- 1: Spitzer
- 2: Spitzergehäuse
- 4: Führungskanal
- 6: Stift-Einführungsseite
- 8: Spitzergehäuse-Rückseite
- 10: Spitzergehäuse-Oberseite
- 12: Spitzmesser
- 14: Freiraum
- 16: Formkante
- 18: Minenformer
- 20: migrierender Stoff
- 22, 24: Beschichtung

## Patentansprüche

1. Spitzer (1) mit einem Spitzergehäuse (2) und mit Formgebungsmitteln, welche mit einem Reinigungsmittel zur Desinfektion eines zu spitzenden medizinischen oder pharmazeutischen Stiftes versetzt sind.

2. Spitzer (1) nach Anspruch 1, bei dem das Reinigungsmittel als Zusatzstoff dem Basisstoff einer Anzahl von Komponenten der Formgebungsmittel beigemischt ist.

3. Spitzer (1) nach Anspruch 1 oder 2, bei dem das Reinigungsmittel als ein- oder mehrlagige Beschichtung (22, 24) auf einer Anzahl von Komponenten der Formgebungsmittel aufgebracht ist.

4. Spitzer (1) nach Anspruch 3, bei dem die Beschichtung (22) Polytetrafluorethylen aufweist.

5. Spitzer (1) nach Anspruch 3 oder 4, bei dem die Beschichtung (24) im Bereich ihrer Oberfläche eine Lotusstruktur aufweist.

6. Spitzer (1) nach einem der Ansprüche 2 bis 5, bei dem der Zusatzstoff bzw. die Beschichtung (22, 24) einen migrierenden Stoff (20) umfasst.

7. Spitzer (1) nach einem der vorhergehenden Ansprüche, bei dem die Formgebungsmittel als Komponenten ein Spitzmesser (12) und einen Minenformer (18) umfassen.

8. Spitzer (1) nach einem der vorgehenden Ansprüche, bei dem das Spitzergehäuse (2) mit dem Reinigungsmittel versetzt ist.

## Claims

1. Sharpener (1) with a sharpener housing (2) and with shaping means which are treated with a cleaning agent for disinfecting a medical or pharmaceutical pencil which is to be sharpened.

2. Sharpener (1) according to Claim 1, wherein the cleaning agent is added, as additive, to the basic material of a number of components of the shaping means.

3. Sharpener (1) according to Claim 1 or 2, wherein the cleaning means is applied, as a coating (22, 24) with one or more layers, to a number of components of the shaping means.

4. Sharpener (1) according to Claim 3, wherein the coating (22) comprises polytetrafluoroethylene.

5. Sharpener (1) according to Claim 3 or 4, wherein the coating (24) has a lotus structure in the region of its surface.

6. Sharpener (1) according to one of Claims 2 to 5, wherein the additive or the coating (22, 24) comprises a migrating material (20).

7. Sharpener (1) according to one of the preceding claims, wherein the shaping means comprise components which are a sharpening blade (12) and a core sharpener (18).

8. Sharpener (1) according to one of the preceding claims, wherein the sharpener housing (2) is treated with the cleaning agent.

## Revendications

1. Taille-crayon (1) comportant un boîtier (2) et des moyens de formage qui sont imprégnés d'un agent de nettoyage pour désinfecter un crayon médical ou pharmaceutique à tailler.

2. Taille-crayon (1) selon la revendication 1, dans lequel l'agent de nettoyage est ajouté à titre d'additif à la substance de base, comportant un certain nombre de composantes, des moyens de formage.

3. Taille-crayon (1) selon l'une ou l'autre des revendications 1 et 2, dans lequel l'agent de nettoyage est appliqué sous forme de revêtement monocouche ou multicouche (22, 24) sur un certain nombre de composants des moyens de formage.

4. Taille-crayon (1) selon la revendication 3, dans lequel le revêtement (22) comprend du polytétrafluoroéthylène.

5. Taille-crayon (1) selon l'une ou l'autre des revendications 3 et 4, dans lequel le revêtement (24) présente dans la zone de sa surface une structure analogue à celle du lotus.

6. Taille-crayon (1) selon l'une des revendications 2 à 5, dans lequel l'additif ou le revêtement (22, 24) contient une substance migrante (20).

7. Taille-crayon (1) selon l'une des revendications précédentes, dans lequel les moyens de formage comprennent comme composants un couteau à tailler (12) et un élément de formage de mine (18).

8. Taille-crayon (1) selon l'une des revendications précédentes, dans lequel le boîtier (2) du taille-crayon est imprégné de l'agent de nettoyage.
